# EUROPÄISCHE PATENTSCHRIFT

(11) **EP 1 737 516 B1**
(45) Veröffentlichungstag und Bekanntmachung des Hinweises auf die Patenterteilung: **11.03.2009**
(21) Anmeldenummer: 05740628.2
(22) Anmeldetag: 05.04.2005
(51) Int. Cl.: A61M 5/168

(54) **VORRICHTUNG ZUR INTRAPERITONEALEN APPLIKATION EINES OZON ODER OZONISIERTEN SAUERSTOFF AUFWEISENDEN FLUIDS IN EINEN MENSCHLICHEN ODER TIERISCHEN KÖRPER**
DEVICE FOR THE INTRAPERITONEAL APPLICATION OF A FLUID COMPRISING OZONE OR OZONISED OXYGEN, IN A HUMAN OR ANIMAL BODY
DISPOSITIF D'ADMINISTRATION PAR VOIE INTRAPERITONEALE D'UN FLUIDE CONTENANT DE L'OZONE OU DE L'OXYGENE OZONISE

(30) Priorität: 07.04.2004 DE 102004017599
(43) Veröffentlichungstag der Anmeldung: 03.01.2007
(73) Patentinhaber: PHILIPPS-UNIVERSITÄT MARBURG, 35037 Marburg (DE)
(72) Erfinder: SCHULZ, Siegfried, 35096 Weimar-Niederwalgen (DE)
(74) Vertreter: Walther, Walther & Hinz GbR
(86) Internationale Anmeldenummer: PCT/DE2005/000597
(87) Internationale Veröffentlichungsnummer: WO 2005/097236

(56) Entgegenhaltungen:
- EP-A- 0 589 356
- EP-A- 0 596 260
- DE-A1- 3 940 389
- US-A1- 2003 065 309

## Beschreibung

Die vorliegende Erfindung betrifft eine Vorrichtung zur intraperitonealen Applikation eines Ozon oder ozonisierten Sauerstoffs aufweisenden Fluids an einen oder in einen in einem menschlichen oder tierischen Körper befindlichen Tumor.

Aus der US 2003/0065309 A1 ist eine Vorrichtung zur Applikation eines Fluids in einem menschlichen und tierischen Körper bekannt, bei dem ein Katheter angelegt wird, um eine Emulsion aus einem Fluidspeicher heraus in den Wirbelsäulenkanal zu applizieren. Hiermit werden Ischias oder andere Krankheiten behandelt. Dabei kann die Emulsion auch Gase, wie zum Beispiel Sauerstoff oder Kohlendioxid beinhalten.

Aus der EP 0 596 260 A1 ist ein Gerät zur medizinischen Ozontherapie durch Transfusion oder Infusion bekannt, mit dem eine Ozon-Eigenblutbehandlung möglich ist.

Es ist wissenschaftlich erwiesen, dass Ozon Bakterien abtöten kann, weshalb Ozon oder ozonisierter Sauerstoff zur therapeutischen Behandlung auch in Menschen oder Tieren eingesetzt wird. Dabei kann eine unsachgemäße Anwendung von Ozon aber auch Gesundheitsschäden verursachen, weshalb Ozon bisher nur exkorporal, in Körperlumen, als mit Ozon angereichertem Blut oder als hyperbare Ozontherapie eingesetzt wurde. Eine Vorrichtung zur Verwendung der hyperbaren Ozontherapie ist beispielsweise aus dem DE 43 40 730 C2 bekannt.

Auch ist es wissenschaftlich erwiesen, dass die bakterien- und gewebetötende Wirkung von Ozon auch zur Abtötung ganzer Tumore führt, wenn nur genügend Ozon appliziert wird.

Es versteht sich, dass unter Tumor auch Mestastasen, Sarkome oder andere Geschwülste zu verstehen sind, insbesondere wenn diese bösartig sind.

Davon ausgehend liegt der vorliegenden Erfindung die Aufgabe zu Grunde, die vorteilhaften Wirkungen von Ozon auch zur Bekämpfung von Tumoren zu nutzen, ohne dass gesundheitsbeeinträchtigende Nebenwirkungen entstehen.

Dabei liegt der vorliegenden Erfindung die Erkenntnis zugrunde, dass die schädlichen Wirkungen des Ozon nicht oder nur stark vermindert eintreten, wenn das Ozon in unmittelbarer Nähe des Tumors oder sogar ins Innere des Tumors appliziert wird.

Desweiteren liegt der vorliegenden Erfindung die Erkenntnis zugrunde, dass die schädlichen Wirkungen des Ozon nicht oder nur stark vermindert eintreten, wenn das Ozon oder der ozonisierte Sauerstoff mit einem minimalen Druck appliziert wird.

Als technische Lösung der Aufgabe wird erfindungsgemäß eine Vorrichtung zur Applikation eines Ozon oder ozonisierten Sauerstoff aufweisenden Fluids in einen menschlichen oder tierischen Körper gemäß den Merkmalen des Anspruches 1 vorgeschlagen. Vorteilhafte Weiterbildungen dieser Vorrichtung sind den Unteransprüchen zu entnehmen.

Eine nach dieser technischen Lehre ausgebildete Vorrichtung hat den Vorteil, dass durch die intraperitoneale Applizierung (Applizierung in die freie Bauchhöhle) des Fluids das Ozon oder der ozonisierte Sauerstoff unmittelbar mit dem Tumor in Kontakt tritt und so seine Wirkung direkt entfalten kann. Dieser Effekt wird noch verstärkt, wenn das Fluid in die direkte Umgebung des Tumors oder sogar in den Tumor selbst appliziert wird.

In einer bevorzugten Verwendung wird das Fluid mit einem sehr geringen Druck zwischen 20 mbar und 50 mbar appliziert. Dies hat den Vorteil, dass das Fluid sehr sanft Kontakt sowohl mit dem Kranken, als auch mit dem gesunden Gewebe aufnimmt. Überraschenderweise haben sich bei den Versuchstieren trotz intensiver und lang anhaltender Applikation entgegen der bisher vorherrschenden Meinung keine gesundheitsschädigenden Nebenwirkungen eingestellt.

In noch einer anderen Verwendung wurden bis zu 100 ml, vorzugsweise 80 ml, je kg Körpergewicht appliziert, ohne dass sich ernst zu nehmende Nebenwirkungen ergaben. Dabei wurde das Fluid vorteilhafterweise in Intervallen appliziert, insbesondere in 3 Einheiten über den Tag verteilt.

Die Ausgestaltung einer entsprechenden Vorrichtung mit einem geringen Arbeitsdruck von 20 mbar bis 60 mbar und mit einem Feinmanometer erleichtert dem behandelnden Arzt die Behandlung, da er nun das Fluid sehr einfach und bequem mit dem gewünschten Druck applizieren kann. Ein weiterer Vorteil besteht darin, dass durch das Feinmanometer eine exakte und den Patienten entsprechende, individuelle Einstellung des Druckes möglich ist. Ein weiterer Vorteil besteht darin, dass auch eine exakte Kontrolle der tatsächlichen Druckverhältnisse durchführbar ist, um die Behandlung optimal überwachen zu können.

Durch die Warnfunktion und insbesondere durch die Abschaltfunktion sind Sicherheitseinrichtungen geschaffen, die eine versehentliche Applikation mit einem unerwünschten Druck verhindern. Folglich wird hierdurch die Behandlungssicherheit erhöht.

In einer besonders bevorzugten Ausführungsform ist ein Potentiometer zum Nullabgleich vorgesehen. Dies hat den Vorteil, dass hiermit atmosphärische Druckschwankungen kompensiert werden können, um eine präzise Druckeinstellung und Druckkontrolle zu ermöglichen.

Weitere Vorteile der erfindungsgemäßen Verwendung und der erfindungsgemäßen Vorrichtung ergeben sich aus den nachstehend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

In einem hier nicht zeichnerisch dargestellten Ausführungsbeispiel ist in einem Gehäuse ein ca. 8 I fassender und als Kolbenzylinder ausgeführter Fluidspeicher zusammen mit einem Feinmanometer untergebracht. Des weiteren ist in dem Gehäuse eine Pumpe vorgesehen, die das Fluid mit einem Druck zwischen 20 mbar und 60 mbar aus dem Fluidspeicher in eine Kanüle pumpt. Diese Kanüle ist zur intraperitonealen Applikation geeignet und mit einem Endoskop ausgerüstet, so dass die Kanüle in der Bauchhöhle zielgerichtet geführt und zum Tumor geleitet werden kann.

Des weiteren ist im Gehäuse eine Warnvorrichtung vorgesehen, die einen akustisches, optisches oder elektronisches Signal auslöst sobald der Arbeitsdruck 40 mbar überschreitet. Ist dies der Fall, so kann der behandelnde Arzt eine Überprüfung der Behandlung vornehmen. Übersteigt der Arbeitsdruck 50 mbar, so schaltet die Abschaltvorrichtung die Fluidzufuhr automatisch ab, um eine gesundheitliche Beeinträchtigung des Patienten zu vermeiden.

Am Feinmanometer ist ein Potentiometer angeschlossen, um vor Beginn der Behandlung den Umgebungsdruck zu messen und um das Feinmanometer auf den Umgebungsdruck einzustellen. Hierdurch wird eine exakte und im gleiche Ausgabe des Fluides erfolgen.

Dabei hat es sich als vorteilhaft erwiesen die aktuellen Druck über eine Anzeige gut sichtbar zu machen.

## Patentansprüche

1. Vorrichtung zur intraperitonealen Applikation eines Ozon oder ozonisierten Sauerstoff enthaltenden Fluids an einen oder in einen in einem menschlichen oder tierischen Körper befindlichen Tumor, mit einem Fluidspeicher, mit einer Vorrichtung zur Abgabe des Fluids aus dem Fluidspeicher und mit einem Feinmanometer zur Erfassung des aktuellen Druckes,
**dadurch gekennzeichnet,**
**dass** im Fluidspeicher ein Ozon oder ozonisierten Sauerstoff aufweisendes Fluid gespeichert ist, dass die Vorrichtung zur Abgabe des Ozon oder ozonisierten Sauerstoff aufweisendes Fluid das Fluid bei einem Arbeitsdruck von 20mbar bis 60 mbar aus dem Fluidspeicher abgibt, und dass das Manometer ein Feinmanometer mit einer Auflösung zwischen 0,1 mbar und 3 mbar, vorzugsweise 1 mbar ist.

2. Vorrichtung nach Anspruch 1,
**gekennzeichnet durch**
eine Warnvorrichtung, welche bei einem Arbeitsdruck von 40 mbar oder mehr ein Warnsignal ausgibt.

3. Vorrichtung nach wenigstens einem der vorangehenden Ansprüche,
**gekennzeichnet durch**
eine Abschaltvorrichtung, welche bei einem Arbeitsdruck von 50 mbar oder mehr die Abgabe des Fluids stoppt.

4. Vorrichtung nach wenigstens einem der vorangehenden Ansprüche,
**dadurch gekennzeichnet,**
**dass** der Fluidspeicher mindestens 5 l, vorzugsweise 8 l des Fluids speichert.

5. Vorrichtung nach Anspruch 4,
**dadurch gekennzeichnet,**
**dass** der Fluidspeicher als Kolbenzylinder ausgebildet ist.

6. Vorrichtung nach wenigstens einem der vorangehenden Ansprüche,
**dadurch gekennzeichnet**
**dass** am Feinmanometer ein Potentiometer angeschlossen ist, mit dem ein Nullabgleich des Feinmanometers durchführbar ist.

7. Vorrichtung nach wenigstens einem der vorangehenden Ansprüche,
**gekennzeichnet durch**
eine intraperitoneale Kanüle.

8. Vorrichtung nach Anspruch 7,
**dadurch gekennzeichnet,**
**dass** die Kanüle mit einem Endoskop versehen ist.

9. Vorrichtung nach wenigstens einem der vorangehenden Ansprüche,
**gekennzeichnet durch**
eine Anzeige zur Sichtbarmachung des aktuellen Arbeitsdruckes.

## Claims

1. A device for intraperitoneal application of a fluid containing ozone or ozonized oxygen onto or into a tumor located in a human or animal body, with a fluid storage container, with a device for delivering said fluid from the fluid storage container and with a precision manometer for measuring the current pressure,
**characterized in**
**that**, in said fluid storage container there is stored a fluid comprising ozone or ozonized oxygen, that said device for delivering fluid comprising the ozone or ozonized oxygen delivers said fluid from said fluid storage container at a working pressure of 20 mbar to 60 mbar and that said manometer is a precision manometer with a resolution ranging between 0.1 mbar and 3 mbar, preferably of 1 mbar.

2. The device as set forth in claim 1,
**characterized by**
a warning device which emits a warning signal at a working pressure of 40 mbar or more.

3. The device as set forth in any one of the afore mentioned claims,
**characterized by**
an interrupting mechanism that stops the release of the fluid at a working pressure of 50 mbar or more.

4. The device as set forth in any one of the afore mentioned claims,
**characterized in**
**that** the fluid storage container stores at least 5 l, preferably 8 l, of the fluid.

5. The device as set forth in claim 4,
**characterized in**
**that** the fluid storage container is realized as a piston cylinder.

6. The device as set forth in any one of the afore mentioned claims,
**characterized in**
**that** a potentiometer, with which a zero adjustment of the precision manometer is possible, is connected to said precision manometer.

7. The device as set forth in any one of the afore mentioned claims,
**characterized by**
an intraperitoneal cannula.

8. The device as set forth in claim 7,
**characterized in**
**that** the cannula is provided with an endoscope.

9. The device as set forth in any one of the afore mentioned claims,
**characterized by**
a display for making visible the current working pressure.

## Revendications

1. Dispositif d'application par voie intrapéritonéale d'un fluide contenant de l'ozone ou de l'oxygène ozonisé sur ou dans une tumeur située dans le corps d'un homme ou d'un animal, avec un réservoir de fluide, avec un dispositif pour distribuer le fluide contenu dans le réservoir de fluide et avec un manomètre de précision pour saisir la pression actuelle,
**caractérisé en ce**
**qu'**un fluide contenant de l'ozone ou de l'oxygène ozonisé est stocké dans le réservoir de fluide, que le dispositif de distribution du fluide contenant de l'ozone ou de l'oxygène ozonisé distribue le fluide contenu dans le réservoir de fluide à une pression de travail comprise entre 20 mbar et 60 mbar et que le manomètre est un manomètre de précision ayant une résolution comprise entre 0,1 mbar et 3 mbar, de préférence de 1 mbar.

2. Dispositif selon la revendication 1,
**caractérisé par**
un dispositif d'avertissement qui émet un signal d'avertissement lorsque la pression de travail se monte à 40 mbar ou plus.

3. Dispositif selon au moins une quelconque des revendications précédentes,
**caractérisé par**
un dispositif interrupteur qui interrompt la distribution du fluide lorsque la pression de travail se monte à 50 mbar ou plus.

4. Dispositif selon au moins une quelconque des revendications précédentes,
**caractérisé en ce**
**que** le réservoir de fluide peut contenir au moins 5 l, de préférence 8 l, de fluide.

5. Dispositif selon la revendication 4,
**caractérisé en ce**
**que** le réservoir de fluide est conformé en forme de piston cylindrique.

6. Dispositif selon au moins une quelconque des revendications précédentes,
**caractérisé en ce**
**qu'**un potentiomètre avec lequel il est possible de remettre le manomètre de précision à zéro est raccordé au manomètre de précision.

7. Dispositif selon au moins une quelconque des revendications précédentes,
**caractérisé par**
une canule intrapéritonéale.

8. Dispositif selon la revendication 7,
**caractérisé en ce**
**que** la canule est munie d'un endoscope.

9. Dispositif selon au moins une quelconque des revendications précédentes,
**caractérisé par**
un afficheur destiné à visualiser la pression de travail actuelle.
